Europäisches Patentamt

European Patent Office

Office européen des brevets

(19)

(11) Publication number: **0 542 287 A2**

## (12) EUROPEAN PATENT APPLICATION

(21) Application number: **92119416.3**

(22) Date of filing: **13.11.92**

(51) Int. Cl.5: **C12P 21/08**, C12N 5/20,
G01N 33/577, //C12N15/60

(30) Priority: **14.11.91 JP 327041/91**
**10.08.92 JP 234278/92**

(43) Date of publication of application:
**19.05.93 Bulletin 93/20**

(84) Designated Contracting States:
**BE CH DE FR GB IT LI NL SE**

(71) Applicant: **IDEMITSU KOSAN COMPANY LIMITED**
**1–1, Marunouchi 3–chome Chiyoda–ku**
**Tokyo 100(JP)**

(72) Inventor: **Irie, Shinji, c/o IDEMITSU KOSAN CO. LTD.**
**1280, Kamiizumi**
**Sodegaura–shi, Chiba 299–02(JP)**
Inventor: **Nakayama, Koji, c/o IDEMITSU KOSAN CO. LTD.**
**1280, Kamiizumi**
**Sodegaura–shi, Chiba 299–02(JP)**
Inventor: **Wadama, Kazuko, c/o IDEMITSU KOSAN CO. LTD.**
**1280, Kamiizumi**
**Sodegaura–shi, Chiba 299–02(JP)**

(74) Representative: **Türk, Gille, Hrabal, Leifert**
**Brucknerstrasse 20**
**W–4000 Düsseldorf 13 (DE)**

(54) Anti–human ornithine decarboxylase antibody.

(57) Antibodies specific to human ornithine decarboxylase and hybridomas producing the same, as well as methods for measuring human ornithine decarboxylase and methods for detecting cancer cells producing human ornithine decarboxylase are disclosed. The present invention provides a polyclonal antibody which specifically binds to human ornithine decarboxylase and a monoclonal antibody which specifically binds to human ornithine decarboxylase. The present invention also provides a hybridoma producing the monoclonal antibody specific to human ornithine decarboxylase.

EP 0 542 287 A2

FIG. 1

## BACKGROUND OF THE INVENTION

### I. Field of the Invention

This invention relates to an anti–human ornithine decarboxylase antibody and to a method of measuring human ornithine decarboxylase using the same. The antibody of the present invention is useful for diagnosis of cancers.

### II. Description of the Related Art

Ornithine decarboxylase (which is hereinafter also referred to as "ODC") is an incipient enzyme of the biosynthesis system of polyamines (Pegg, A.E., Biochem. J., Vol. 234, p.249, 1986). It is known that ODC rapidly exhibits activity change upon stimulation with drugs and that this relates to cell growth and carcinogenesis (Feinstein, S. C. et al., Proc. Natl. Acad. Sci. USA, Vol. 82, 5761, 1985; Katz A. et al., Mol. Cell. Biol., Vol. 7, 2641, 1987; Lau, L. et al., Proc. Natl. Acad. Sci. USA, Vol. 84, 1182, 1987). Further, it is known that ODC level increases when cells grow, so that ODC can be an index showing the degree of biological malignance of tumors (Shinzo MURAKAMI et al., Japanese Journal of Gastroenterology, Vol. 86, 1260, 1989; Kazuo NAKANISHI et al., Journal of Clinical and Experimental Medicine (IGAKU NO ATUMI), Vol. 153, p.709, 1990; Osami YAMAMOTO, Nichigaikaishi, Journal of Japan Surgical Society, Vol. 90, 1049, 1989; Narisawa, T. et al., Cancer, Vol. 63, 1572, 1989).

ODC activity is usually measured by absorbing $^{14}CO_2$ generated by decarboxylization of $^{14}C$–labelled ornithine in a carbon dioxide absorber such as protosol and measuring the absorbed $^{14}CO_2$ (Russell D. et al., Proc. Natl. Acad. Sci. USA, Vol. 60, 1420, 1968). However, with this method, since a radio active gas is employed, the operation is troublesome and the error is large. On the other hand, radio immunoassays for measuring ODC have been proposed. These include a method in which $^3H$–DFMO–labelled ODC is used (Seely, J. E. et al., J. Biol. Chem., Vol. 258, 2496, 1983) and a competitive method in which $^{125}I$–labelled ODC is used (Isomma,V.V. et al., J. Biol. Chem., Vol. 258, 6735, 1983). The former has a drawback in that its sensitivity is not high and the latter has a drawback in that the troublesome preparation of $^{125}I$–labelled ODC is required.

To overcome these drawbacks, an enzyme immunoassay (Nishiyama M et al., J. Immunoassay, Vol. 10, p.19, 1989) and a method employing a monoclonal antibody (CA1215010, Japanese Laid–open Patent Application (Kokai) No. 2–176564) have been proposed. However, since it is difficult to obtain human ODC in an amount sufficient to immunize an animal, the ODC employed in the conventional methods is derived from animals other than human, such as mouse and rat. It is expected that the ODCs of animals have antigenicities different from that of human, so that it is difficult to apply the conventional anti–ODC antibodies to human clinical applications which require high sensitivity.

## SUMMARY OF THE INVENTION

Accordingly, an object of the present invention is to provide an antibody whose corresponding antigen is human ODC. Another object of the present invention is to provide a method for measuring human ODC by using the antibody of the present invention.

As a result of intensive study, the present inventors succeeded in preparing human ODC in a large amount by a genetic engineering method, and succeeded in obtaining an anti–human ODC polyclonal antibody and anti–human ODC monoclonal antibodies, thereby completing the present invention.

That is, the present invention provides a polyclonal antibody which specifically binds to human ornithine decarboxylase. The present invention also provides a monoclonal antibody which specifically binds to human ornithine decarboxylase. The present invention further provides a hybridoma which produces the monoclonal antibody of the present invention. The present invention still further provides a method for measuring human ornithine decarboxylase by an immunoassay utilizing the specific binding between the antibody of the present invention and human ornithine decarboxylase. The present invention still further provides a method for detecting cancer cells producing human ornithine decarboxylase, comprising immunostaining the human ornithine decarboxylase with the antibody of the present invention.

By the present invention, anti–human ODC polyclonal antibody and an anti–human ODC monoclonal antibody, as well as a method for measuring human ODC were first provided. Unlike the conventional antibodies, since the corresponding antigen of the antibody of the present invention is human ODC, the antibody enables the detection of human ODC with high sensitivity in, for example, diagnosis of cancers.

3

## BRIEF DESCRIPTION OF THE DRAWINGS

Fig. 1 shows the construction of a vector for expressing modified protein A, which was used in the preparation process of human ODC;

Fig. 2 shows the construction of a recombinant plasmid containing cDNA of human ODC, which was used in the preparation process of human ODC;

Fig. 3 shows the construction of a recombinant plasmid encoding a fusion protein of glutathione S transferase and human ODC, which was used in the preparation process of human ODC;

Fig. 4 shows the results of Western blotting using the antibody of the present invention;

Fig. 5 shows the results of Western blotting showing the reaction between the antibody of the present invention and extract of mouse kidney induced by testosterone;

Fig. 6 shows a calibration curve obtained by an immunoassay using the antibody of the present invention and standard samples; and

Fig. 7 shows the base sequence of the cDNA of human ODC.

## DETAILED DESCRIPTION OF THE PREFERRED EMBODIMENTS

The method for preparing the polyclonal antibody and monoclonal antibody of the present invention will now be described in order. However, it should be noted that the following is a preferred embodiment and the present invention is not restricted thereto.

(1) Preparation of Immunogen

(A) Human cDNA

To prepare human ODC by a genetic engineering process, human cDNAs are first obtained. The human cDNAs can be obtained according to a conventional method by extracting mRNAs from a human tissue (such as kidney, liver, heart, placenta, pancreas, large intestine or the like) or human cultured cells, and by synthesizing cDNAs using a reverse transcriptase employing the mRNAs as templates. Alternatively, since human cDNA libraries are commercially available (for example, λgt10 libraries from kidney, liver, heart, plancenta, pancreas or large intestine or various human cultured cells commercially available from CLONTECH), such commercially available human cDNA libraries may also be employed.

(B) Gene Amplification Reaction (PCR method)

(i) Primer

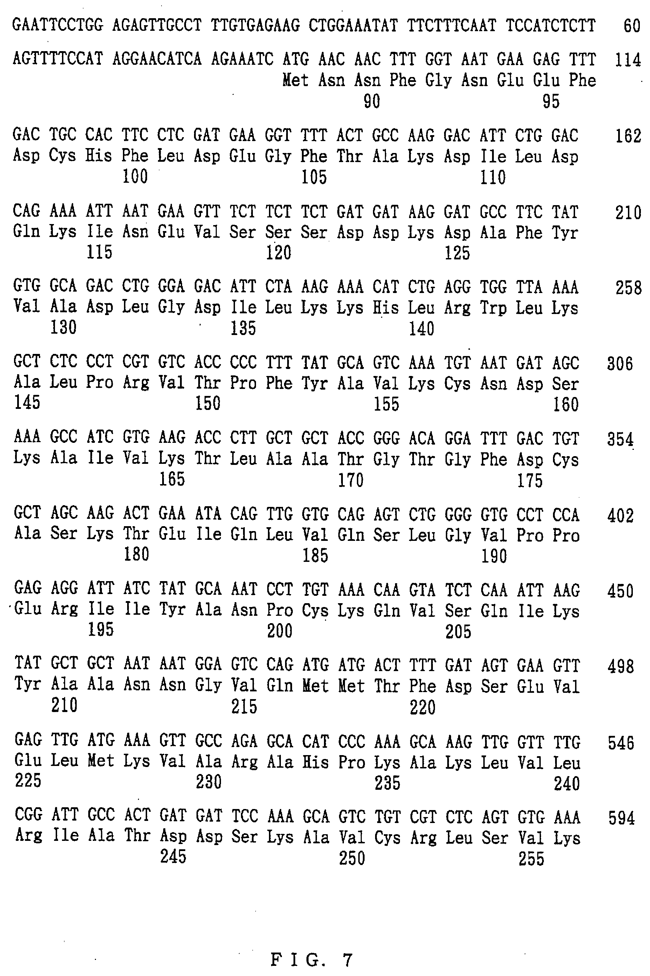

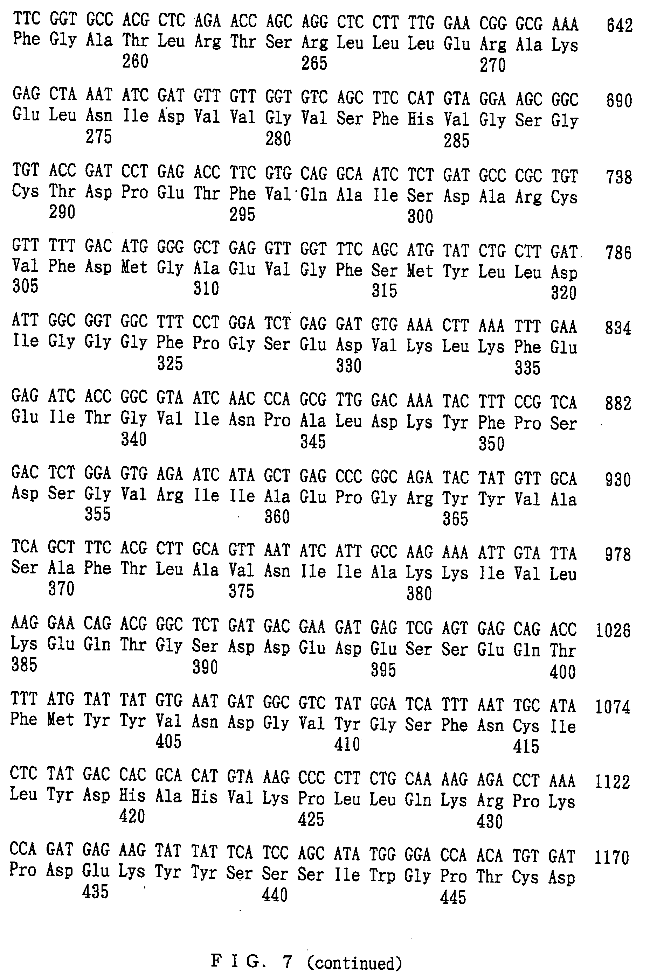

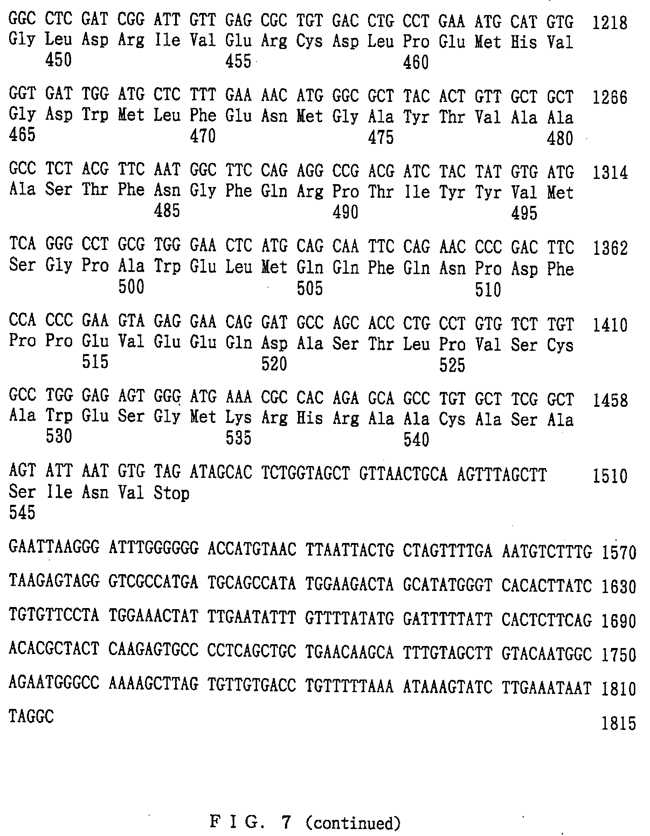

The base sequence of the cDNA of human ODC has been determined by Hickok et al (Hickok, N.J. et al., DNA, Vol. 6, 179, 1987). Thus, two points in the cDNA are optionally selected and the primers are chemically synthesized based on the base sequence by using a DNA synthesizer (e.g., DNA Synthesizer 391−02 commercially available from Applied Biosystems). The two points should be selected so that sufficiently long DNA fragment is amplified such that the polypeptide encoded by the replicated DNA fragment has immunogenecity. The primers may preferably have a length of 20 − 30 oligonucleotides. Restriction sites may be added to the 5'−ends of the primers so that the amplified DNA fragment can easily be ligated to an expression vector. The base sequence of the cDNA of human ODC determined by Hickok et al is shown in Fig. 7.

(ii) Conditions of PCR

A reagent kit of PCR is commercially available from Perkin−Elmer−Cetus, so that the PCR can be carried out by following the instructions of the kit. Alternatively, the PCR may be performed according to the following conditions:

Composition of Reaction Mixture (in 100 $\mu$l)

| DNA | 0.1 − 1 $\mu$g |
|---|---|
| Primer | 25 − 100 pmol each |
| dNTP | 200 $\mu$M each |
| Tris − HCl (pH 8.3) | 10 mM |
| KCl | 50 mM |
| MgCl$_2$ | 1.5 mM |
| gelatin | 0.01 wt% |
| Taq polymerase | 2.5 − 5 units |

First Step: 95ºC, 30 seconds to 1 minute
Second Step: 37 − 55ºC, 30 seconds to 2 minutes
Third Step: 70 − 74ºC, 1 − 3 minutes
After the third step, the process returns to the first step, and the cycle of first, second and third step is repeated 25 − 40 times.

(iii) Preparation of Amplified cDNA

The DNA amplified by PCR can be recovered by, for example, subjecting the PCR product to 0.6 − 2 wt% agarose gel electrophoresis, cutting out the DNA band detected by ethidium bromide staining and subjecting the cut out band to a routine process for recovering DNA (Maniatis T. et al., "Molecular Cloning", Cold Spring Harbor Lab. NY).

(C) Ligation with Gene Expression Vector and Construction of Recombinant Plasmid

(i) Expression Vector

As the expression vector, those generally employed in genetic engineering, that is, those containing a promoter for expressing a gene, an SD sequence, restriction sites for inserting a gene, and a terminator may be employed. The expression vectors by which the desired gene product is expressed as a fusion protein with a protein such as protein A which can be purified by affinity chromatography are especially preferred because the desired gene product may easily be purified. Such expression vectors are commer − cially available and examples thereof include pKK233 − 3, pRIT2T and pGEX − 3X commercially available from Pharmacia.

(ii) Ligation of cDNA and Construction of Recombinant Plasmid

The DNA prepared in (1)(B) is ligated with an expression vector cut by a restriction enzyme. A number of restriction enzymes are commercially available, and the ligation reaction can be carried out using a commercially available enzyme or kit (e.g., ligation kit commercially available from Takara Shuzo Co., Ltd., Kyoto, Japan). The resulting reaction product is used for transforming competent E. coli cells, and the desired recombinant plasmid can be obtained by selecting E. coli cells carrying the recombinant plasmid. These operations are ordinary operations employed in genetic engineering and described in, for example, Maniatis et al., (supra).

(D) Culture of Transformants

The E. coli cells containing the recombinant plasmid are cultured in a medium such as LB medium until middle of logarithmic phase and an induction treatment for expressing the desired gene (e.g., addition of 1 mM IPTG when the expression is carried out under the control of tac promoter, or raising the culture temperature to 42ºC when λP$_R$ promoter is employed) is performed, followed by additional culturing for another 2 − 4 hours.

(E) Separation and Purification of Human ODC

The transformants are collected by centrifugation and the cells are disrupted by an ultrasonic treatment or the like. In cases where the gene product forms an inclusion body, the gene product is solubilized by urea after centrifugation. The human ODC may be separated and purified by adsorption by ion−exchanger (DEAE or the like), gel permeation, electrophoresis and/or by affinity method utilizing a carrier which specifically binds to human ODC. In cases where human ODC is expressed as a fusion protein, the human ODC may be cut out from the fusion protein with a proteolytic enzyme Factor Xa (commercially available from Boehringer Mannheim) and the human ODC may then be purified by adsorption by ion−exchanger (DEAE or the like), gel permeation, electrophoresis and/or by affinity method utilizing a carrier which specifically binds to human ODC. An example of the separation and purification of human ODC is described in the Examples hereinbelow described.

(2) Immunization of Animals and Obtainment of Polyclonal Antibody

An animal may then be immunized with the thus obtained purified human ODC according to a conventional method. The immunization may be performed by, for example, administering the purified human ODC with or without a carrier or a diluent to a warm−blooded animal such as monkey, rabbit, dog, guinea pig, mouse, rat, sheep, goat or chicken via subcutaneous route or other routes. To promote the production of antibody, Freund's complete adjuvant or incomplete adjuvant may be administered together with the immunogen. The immunization is usually carried out by administering the immunogen totally 3 − 6 times with 2 − 6 weeks' interval. The desired polyclonal antibody can be recovered from the blood of the immunized animal.

(3) Obtainment of Monoclonal Antibody

(A) Immunization

A warm−blooded animal such as mouse is immunized in the same manner as described in the preceding section describing the obtainment of the polyclonal antibody.

(B) Cell Fusion

Immunized animals exhibiting the desired antibody titer are selected and spleens or lymphnodes are collected therefrom at 2 − 5 days after the final immunization. Then antibody−producing cells contained therein are fused with myeloma cells (e.g., NS−0, NS−1, P3U1 and the like). The cell fusion may be carried out according to a conventional method such as the method by Kohler and Milstein (Nature, Vol. 256, p.495, 1975). The preferred ratio of the antibody−producing cells (spleen cells) to the myeloma cells may be 1:1 to 20:1. The cell fusion may be efficiently carried out by adding polyethylene glycol (PEG) preferably having a molecular weight of 1000 − 6000 in an amount of 10 − 80% by weight, and by incubating the reaction mixture at 30 − 37ºC for 1 − 10 minutes.

(C) Screening

The hybridomas are selected by culturing the cells in HAT medium according to a conventional method. The screening of the desired hybridoma may then be carried out by enzyme immunoassay in which the culture supernatants of the obtained hybridomas are added to a solid phase immobilizing human ODC (e.g., wells of a microplate), then an anti−immunoglobulin antibody (when the antibody−producing cells subjected to the cell fusion are obtained from a mouse, an anti−mouse immunoglobulin antibody is used) is added and then the enzyme label is measured thereby measuring the anti−human ODC monoclonal antibody immobilized to the solid phase.

(D) Cloning

The cloning of the human ODC−producing hybridoma can be carried out by a conventional method such as limiting dilution method or soft agar method.

(E) Production of Monoclonal Antibody

The monoclonal antibody may be produced by culturing the hybridomas in a large scale using a serum − containing medium or a serum − free medium, or by intraperitoneally administering the hybridomas to mice and collecting the monoclonal antibody from the ascites of the mice.

(4) Purification of Antibody

The monoclonal antibody can be purified by a conventional method by using an affinity column containing protein A − Sepharose (trademark) or protein G − Sepharose (trademark) or by high performance liquid chromatography.

The polyclonal antibody and the monoclonal antibody of the present invention may be used for measuring human ODC by an immunoassay utilizing the specific binding between the antibody and human ODC. The type of the immunoassay is not restricted and the antibody may be used in any conventional immunoassay such as enzyme immunoassay or sandwich enzyme immunoassay. A preferred mode is sandwich enzyme immunoassay. That is, a primary antibody according to the present invention is immobilized on a solid carrier such as wells of a microplate and microbeads, and the resultant is then contacted with a test sample. A second antibody according to the present invention whose epitope is different from that of the primary antibody, which is labelled with an appropriate marker is then reacted, and the marker immobilized on the solid phase is then measured. Further, human tissues may be im − munochemically stained with the antibody according to the present invention, thereby detecting cancer cells.

The invention will now be described by way of examples thereof. It should be noted that the examples are presented for the illustration purpose only and should not be interpreted in any restrictive manner.

(1) Construction of ODC − cDNA Expression System

(i) Preparation of ODC − cDNA

Based on the base sequence of ODC − cDNA (shown in Fig. 7), an oligonucleotide having the base sequence at the 5' end portion (23 nucleotides) of the ODC − encoding region to which Bam HI site is added to its 5' end (the overall oligonucleotide has 30 nucleotides) and an oligonucleotide having the base sequence at the 3' end portion (23 nucleotides) of the ODC − encoding region to which Eco RI site is added to its 5' end (the overall oligonucleotide has 30 nucleotides) were synthesized according to a conventional method using a DNA synthesizer (Model 391 − 02, commercially available from Applied Biosystems). The oligonucleotides had the following sequences:

5' side 5' − GGGATCCCCATGAACAACTTTGGTAATGAA − 3'
3' side 5' − GGGAATTCTACACTTAAATACTAGCCGAAG − 3'

These oligonucleotides were purified by an oligonucleotide − purifying cartridge (OPC, commercially avail − able from Applied Biosystems), and were used as primers for PCR.

DNAs from human kidney cDNA library (λgt 10 library, commercially available from Clonetech) were amplified by a conventional method (Maniatis et al., supra). Polymerase chain reaction was carried out using 1 μg of the thus obtained DNAs, 100 pmol each of the primers, DNA amplification reagents kit (GeneAmp, commercially available from Perkin − Elmer − Cetus) and DNA thermal cycler (commercially available from Perkin − Elmer − Cetus). A cycle of thermal denaturation (95°C, 1 minute), annealing (55°C, 2 minutes) and complement chain extension (72°C, 3 minutes) was repeated 25 times. The reaction product was then subjected to 0.6 wt% agarose gel electrophoresis and a single band corresponding to about 1.4 kb was detected. The band was cut out from the gel and DNA was recovered by a conventional method. The obtained DNA was digested with Bam HI and Eco RI and the resultant was subjected to 0.6 wt% agarose gel electrophoresis, followed by recovery of the DNA from the emerged single band, thereby obtaining ODC − cDNA as a Bam HI − Eco RI fragment.

(2) Preparation of ODC − cDNA Expression Plasmid

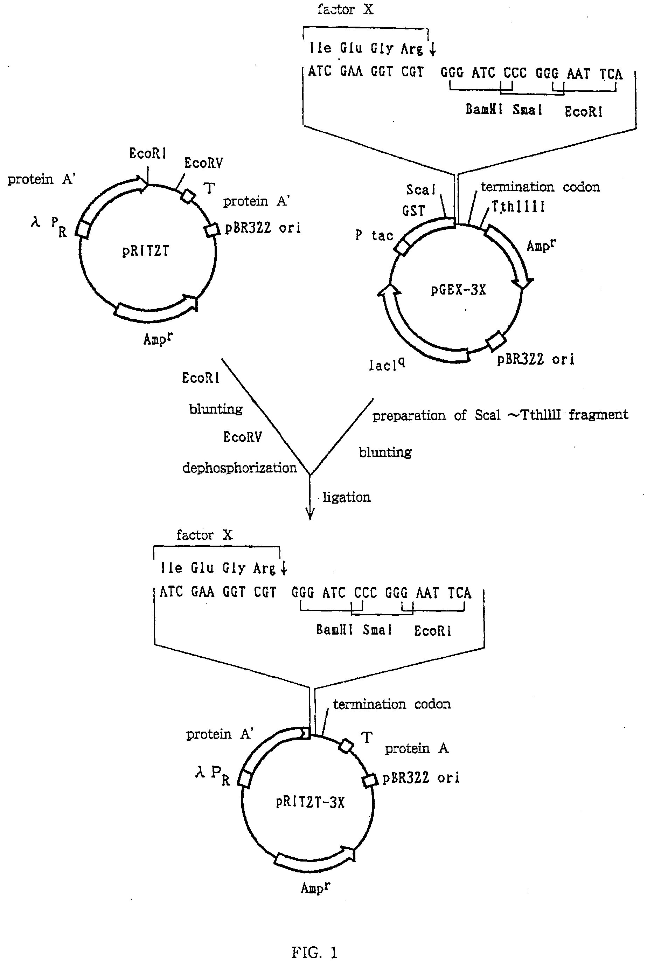

In order to assure the cut off of the protein A region from the fusion protein expressed by a protein A fusion gene vector (pRIT2T, commercially available from Pharmacia), the region encoding the C − terminal of protein A was exchanged with the C − terminal encoding region of glutathione S transferase (GST) fusion gene vector (pGEX − 3X, commercially available from Pharmacia), which can be cut off by protease Factor X, to prepare a modified protein A fusion gene vector (Fig. 1). This vector was used for the expression of ODC – cDNA.

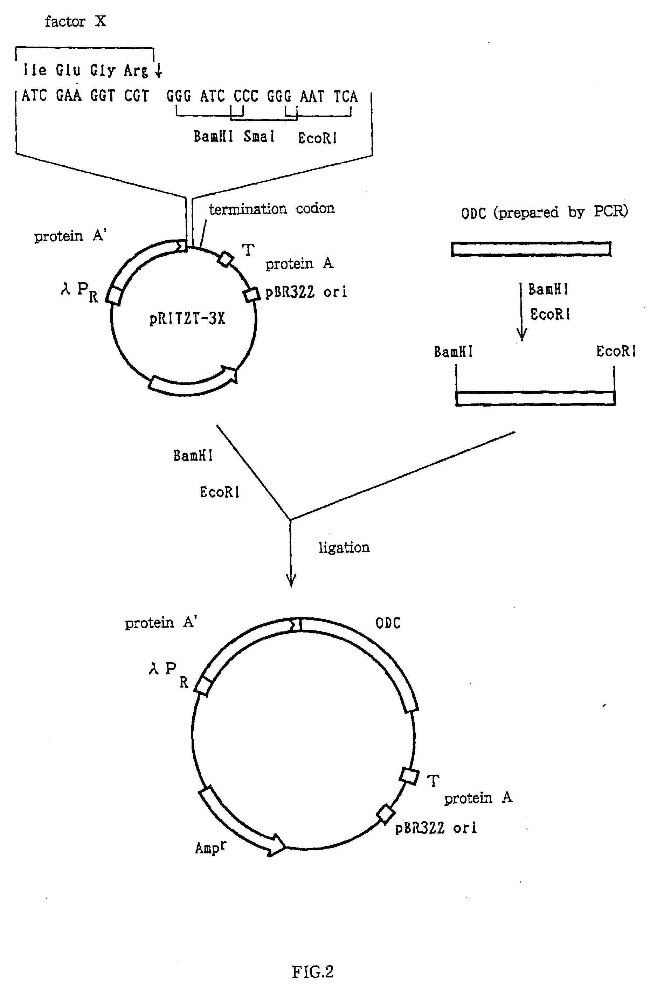

This vector was digested with restriction enzymes Bam HI and Eco RI and the resultant was ligated with the ODC – cDNA fragment prepared in (i). An E. coli N99cl+ (commercially available from Pharmacia) in which the gene expression cannot be induced was transformed with the resulting recombinant vector and the E. coli cells containing the recombinant vector (Fig. 2) having the ODC – cDNA fragment were selected. Plasmids were extracted from the E. coli cells, and E. coli N4830 – 1 (commercially available from Pharmacia) in which the gene expression can be induced was transformed with the thus obtained plasmids. The obtained transformants were cultured in LB medium at 30°C until middle of logarithmic phase ($OD_{600}$ = 0.3), and then cultured at 42°C for 2.5 hours. The crude protein from the thus cultured transformants was subjected to SDS – polyacrylamide gel electrophoresis to detect a band corresponding to about 90 kd, which corresponds to the fusion protein of protein A and ODC.

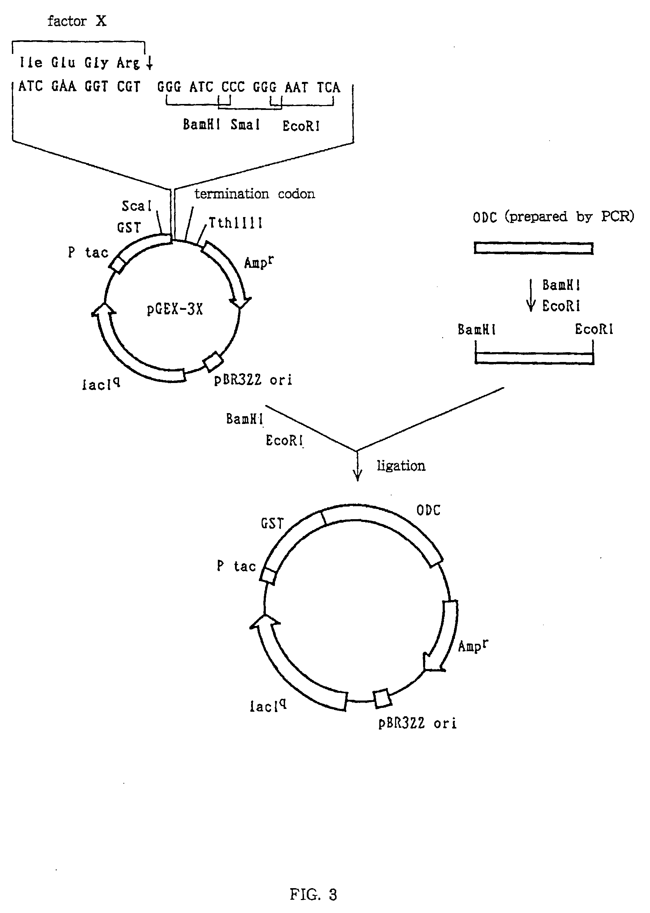

Similarly, pGEX – 3X was ligated with the DNA fragment prepared in (i) and the resultant was used for the transformation of E. coli JM109 to prepare a transformant which produces a fusion protein of glutathione S transferase and ODC (Fig. 3).

(2) Production of ODC

(i) Production of Protein A – ODC Fusion Protein by E. coli

The E. coli N4830 – 1 cells containing the plasmids for expressing the protein A – ODC fusion protein were cultured in LB medium at 30°C to middle to end of logarithmic phase. The culture temperature was then raised to 42°C and the culture was continued for another 4 hours to produce the protein A – ODC fusion protein.

Similarly, the E. coli Y1091 (commercially available from Clonetech) containing the plasmid for expressing GST – ODC fusion protein was cultured in LB medium at 37°Cuntil middle to end of logarithmic phase. Thereafter, IPTG was added to a final concentration of 1 mM and the culture was continued for another 4 hours to produce GST – ODC fusion protein.

(ii) Extraction of Fusion Protein

The culture medium of E. coli described in (i) was centrifuged to collect E. coli cells. The bacterial cells were suspended in buffer A (20 mM Tris – HCl, pH 8.0 + 5 mM EDTA) and then ultrasonically disrupted with a ultrasonicator (SONIFIER 250, commercially available from Branson). The resulting suspension was centrifuged at 1000 x g to obtain the fusion protein as a precipitated fraction.

(iii) Solubilization of Fusion Protein

The precipitated fraction obtained in (ii) was suspended in buffer A and saturated solution of urea was added thereto to a final concentration of 8M. Thereafter, 2 – mercaptoethanol was added to a concentration of 1% (v/v) and the resultant was left to stand at 60°C for 1 – 4 hours to solubilize the precipitate.

(iv) Separation of Fusion Protein

The solution obtained in (iii) was passed through an anion – exchange resin (Toyopearl 650S, commercially available from Tosoh Corporation, Yamaguchi, Japan) equilibrated with buffer B (20 mM Tris – HCl, pH8.0 + 6M urea) and adsorbed components were eluted with a gradient of 0 – 0.3 M sodium chloride. Among the eluted fractions, those containing the fusion protein were combined and concentrated by ultrafiltration (Centriprep 30, commercially available from Amicon). To the resulting concentrate, a denaturing solution (1 wt% SDS, 6 M urea, 1 wt% 2 – mercaptoethanol (final concentrations)) was added, and the resulting mixture was boiled at 100°Cfor 2 minutes. The resultant was subjected to gel permeation through a gel permeation column (Superdex 200, commercially available from Pharmacia) equilibrated with buffer C (20 mM Tris – HCl, pH 8.0 + 0.1 wt% SDS). Among the eluted fractions, those containing the fusion protein were collected and the solvent was replaced with buffer A by ultrafiltration. Since the residual SDS in the resulting solution inhibits Factor Xa, the residual SDS was removed by a surfactant – removing agent (ExtractiGeID, commercially available from Pierce).

(v) Cut off of ODC from Fusion Protein

The fusion protein obtained in (iv) was diluted with a reaction solution (50 mM Tris−HCl, pH 8.0 + 100 mM NaCl + 5 mM CaCl$_2$) so as to attain a final concentration of the fusion protein of 1 mg/ml and a ratio by weight of the fusion protein to Factor Xa of 100:1. The Factor Xa commercially available from Boehringer Mannheim was used. The reaction was carried out at 4°C overnight or at 25°C for 2 − 6 hours.

(vi) Separation of ODC

To the reaction mixture obtained in (v), saturated solution of urea was added to a final concentration of 8 M. To the resultant, 2−mercaptoethanol was added to a final concentration of 1% (v/v) and the resulting mixture was left to stand at 60°C for 1 − 4 hours. The thus obtained solution was passed through an anion exchange resin (Mono Q, commercially available from Pharmacia) equilibrated with buffer D (buffer B + 0.1% 2−mercaptoethanol), and the adsorbed components were eluted with 0 − 0.3 M gradient of sodium chloride. The eluted fractions containing ODC were combined and concentrated by ultrafiltration. The obtained concentrate was passed through a gel permeation column (G3000 SW, commercially available from Tosoh Corporation) equilibrated with buffer E (50 mM Na−Pi, pH6.2 + 6 M urea + 0.5 M NaCl + 2% DMSO), and the eluted fractions containing ODC were combined. The solvent of the eluate was replaced with buffer A to obtain purified ODC.

The ODC originated from protein A − ODC fusion protein was used for immunization, and the ODC originated from GST−ODC fusion protein was used for the evaluation such as screening of antibodies, to eliminate interferences by contaminating proteins other than ODC.

(vii) Confirmation of Purified ODC

To confirm the purified ODC, the amino acid sequence of the N−terminal region of the protein was determined by an automatic gas phase protein sequencer (470A, commercially available from Applied Biosystems). The sequencing was performed by an ordinary method using 500 pmol of sample. The result is shown in Table 1 below.

## Table 1 .

```
Found:   Gly Ile Pro Met Asn Asn Phe Gly Asn Glu Glu Phe
Reference:               Met Asn Asn Phe Gly Asn Glu Glu Phe
                          1               5

Found (continued)        Asp Xaa His Phe Leu Asp Glu Gly
Reference (continued)    Asp Cys His Phe Leu Asp Glu Gly
                          10               15
```

Xaa: not determined

(3) Immunization

To 1 ml of 5 mM phosphate buffer (pH 7.0) containing 100 $\mu$g of ODC obtained in (2), 1 ml of Freund's complete adjuvant was added and the mixture was well mixed. The obtained mixture was subcutaneously administered to a rabbit through 20 points of the skin. Two weeks later, ODC was administered to the rabbit in the same manner as described above except that Freund's incomplete adjuvant was used in place of Freund's complete adjuvant. Another two weeks later, ODC was administered to the rabbit in the same manner as in the second immunization except that the amount of the administered ODC was 200 $\mu$g. The immunization in the same manner as the third immunization was repeated another three times at two weeks intervals. Seven days after the final immunization, the total blood was collected and antiserum was prepared therefrom according to a conventional method.

On the other hand, ODC was intraperitoneally administered together with an adjuvant to female BALB/C mice of 8 weeks old in an amount of 50 μg/mouse. Two weeks later, additional immunization was performed. Further, 3 − 4 days before sacrifice, the antigen alone was administered through the veins in the tails.

(4) Cell Fusion

Three to four days after the final immunization, spleens were removed from the mice immunized as described in (3). The spleens were disrupted by a homogenizer and the spleen cells were suspended in PBS. Then the spleen cells and myeloma cells (SP2/0 − Ag14, commercially available from Dainippon Pharmaceutical Co., Ltd., Osaka, Japan) were mixed at a ratio of 5:1 and the resulting mixture was left to stand for 3 minutes in the presence of 50% polyethylene glycol (PEG4000). After removing the supernatant by centrifuging the resultant at 1200 rpm for 8 minutes, 25 ml of RPMI − 1640 medium was gradually added and the resulting mixture was centrifuged at 1200 rpm for 8 minutes. The cells were then suspended in HAT RPMI − 1640 medium containing 10% fetal calf serum (FCS) and 10% hybridoma cloning factor (HCF) to a population density of $1.75 \times 10^6$ cells/ml. The obtained cell suspension was placed in the wells of seven 96 − well microtiter plates in an amount of 0.2 ml/well. The cells were then cultured in an incubator containing 5% carbon dioxide gas at 37°C. After 7 days from the beginning of the incubation, 0.1 ml/well of the culture medium was replaced with the same volume of HAT RPMI − 1640 medium containing 10% FCS and 10% HCF. After 7 − 10 days from the beginning of the incubation, formation of colonies by growth of hybridomas was observed. Sufficient production of antibodies to the antigen were confirmed in several tens wells by ELISA described below.

(5) Screening

Screening of hybridomas was performed by enzyme immunoassay (ELISA, Immunochemistry, Vol. 8, p871, 1971) as follows: That is, 50 μl/well of PBS containing 5 μg/ml of ODC or 50 μl/well Of homogenized colon cancer cell (0.8 mg/ml of concentration of protein prepared according to (8)) was placed in the wells of a 96 − well microtiter plate and the proteins were adsorbed on the wells by incubation at 4°C for 24 hours or at 30°C for 2 hours. The wells were then washed with PBS containing 0.05% Tween − 20 (PBS − Tween) and 300 μl/well of a blocking solution (Block Ace, commercially available from Dainippon Pharmaceutical, 4 − fold diluted) was added and the resultant was incubated at 4°C for 24 hours or at 30°C for 2 hours. To the wells, 50 μl/well of culture supernatant was added and the resultant was allowed to react at 30°C for 2 hours. After the reaction, the wells were washed wish PBS − Tween, and 100 μl/well of peroxidase − labelled anti − mouse immunoglobulin antibody (commercially available from Cappel, 1000 − fold diluted with PBS containing 0.1% BSA) was added and the resultant was allowed to react at 30°C for 1 hour. After the reaction, the wells were washed with PBS − Tween and 100 μl/well of 0.1 M citrate − phosphate buffer (pH 5.0) containing 0.04% o − phenylenediamine and 0.02% hydrogen peroxide was added to the wells. The resultant was allowed to react at 30°C for 3 − 10 minutes and then 20 − 50 μl/well of 3.6 N sulfuric acid was added to stop the reaction. The absorbances at 492 nm and at 630 nm of the wells were measured by a plate reader (MTP − 120, commercially available from Corona), and the antibody activities were evaluated.

From the wells which exhibited positive antibody activities, 27 hybridomas were selected and cultured in a 24 − well microtiter plate. The antibody activities were again measured and 17 hybridomas were finally selected. Among these, 2 hybridomas (3B − 10 and 4B − 6) were deposited with Fermentation Research Institute of Japan under the Budapest Treaty under accession numbers of FERM BP − 4069 and FERM BP − 4070, respectively.

(6) Cloning of Hybridomas and Determination of Class and Subclass of Monoclonal Antibodies

The 17 hybridomas were then cloned by the limiting dilution method. That is, the hybridomas were suspended in HT − RPMI 1640 medium containing 10% FCS and 10% HCF to a population density of 5 cells/ml or 10 cells/ml, and the suspensions were placed in the wells of 96 − well microtiter plates in an amount of 0.2 ml/well. After incubation at 37°C for 7 − 10 days, the hybridomas forming only one colony in one well and exhibiting positive antibody activities were selected as the desired monoclonal antibody − producing hybridomas. As a result, 14 clones of hybridomas were obtained. The immunoglobulin class and subclass of the monoclonal antibodies produced by the hybridomas were determined by using a mon − oclonal antibody subtyping kit (commercially available from Bio − Rad), which were IgG2a as well as IgG2b (3B−10, 4B−6) and IgM. The cloning operation described above was repeated again and monoclonal antibodies were obtained therefrom.

(7) Purification and Labelling of Antibodies

(i) Separation and Purification of Antibodies

To the antiserum obtained in (3), equivolume of a binding buffer (20 mM Na−Pi, pH 7.0) was added and the resulting mixture was passed through Protein G − Sepharose 4 column (commercially available from Pharmacia) equilibrated with the binding buffer to adsorb IgG. After washing the column with the binding buffer, IgG was eluted with an eluent buffer (100 mM glycine−HCl, pH 2.7). The eluate was concentrated by ultrafiltration (Centriprep−30, commercially available from Amicon), and the obtained concentrate was dialyzed against the binding buffer to obtain purified polyclonal antibody. In the same manner, monoclonal antibodies were purified from the culture supernatants of the hybridomas.

(ii) Labelling of Polyclonal Antibody

Biotin was bound to the polyclonal antibody obtained in (i) using a kit for labelling proteins with biotin (commercially available from Amersham) in accordance with the instructions of the kit.

(8) Examination of Reactivities of Polyclonal Antibody and Monoclonal Antibodies by Western Blotting

(i) Reactivities with Cultured Human Cancer Cells

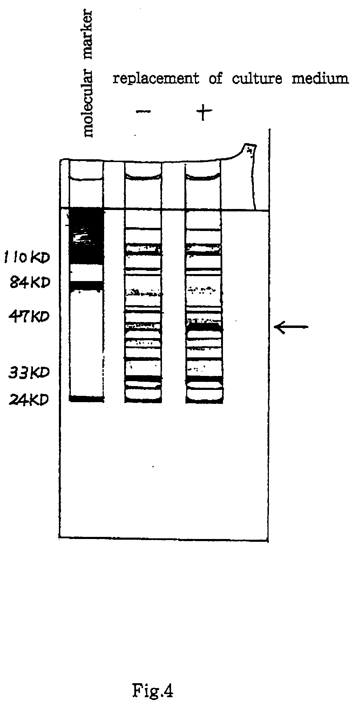

The ODC activity of cultured cells is increased about 10 times by replacing the medium (D'Agostino et al., Gastroenterology, Vol. 97, p888, 1989). Thus, human originated cells (COLO 205, originated from colon cancer, ATCC accession number: CCL−222) were cultured in RPMI 1640 containing 10% FCS at a population density of $2.7 \times 10^5$ cells/ml and the medium was replaced after 3 days from the beginning of the culture. The culture was continued for another 6 hours to prepare cells which were expected to have high ODC activity. As a control, the cells cultured without changing the medium were also prepared. The medium in the flask in which the cells were grown was removed and the cells were treated with PBS containing 0.25% trypsine. The resultant was centrifuged and the precipitate was again washed with PBS, followed by centrifugation. The precipitate was subjected to SDS−polyacrylamide gel electrophoresis using Fast System, commercially available from Pharmacia, the amount of applied protein per lane being 15 μg. After the electrophoresis, the bands were transferred to a polyvinylidene difluoride membrane (Clear Blot P Membrane, commercially available from Ato Co., Ltd). The transferred membrane was tested for the bands which bind to each antibody. As a result, when the polyclonal antibody (purified IgG) is used as a primary antibody, with the cells for which the medium was changed, a specific and dense band was detected at the same position as the recombinant ODC. However, with the cells for which the culture medium was not replaced, a thin band was detected (Fig. 4). Similarly, when the monoclonal antibody is used as the primary antibody, a specific band was detected for the cells for which the culture medium was changed, although it was weaker than in the case of using the polyclonal antibody.

(ii) Reaction with Teststerone−induced Mouse Kidney  Extract

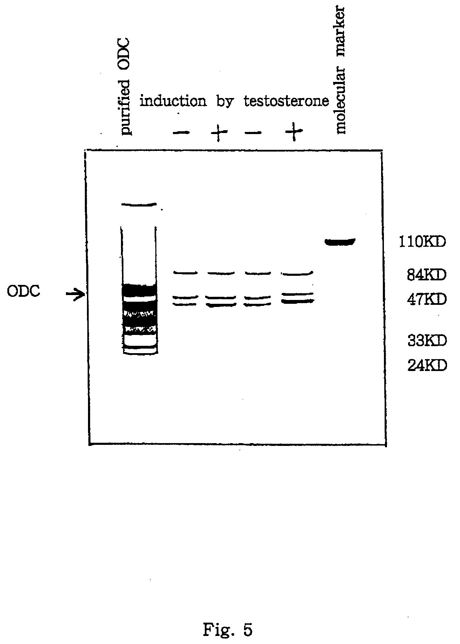

Since the ODC content in mouse kidney is increased by treating the mouse with testosterone (Nishiyama et al., J. Immunoassay, Vol. 10, p.19, 1989 and Biol. Chem. Hoppe−Seyler, Vol. 371, p.363, 1990), male mice (DDY) were treated with testosterone in a conventional manner. One week after, kidneys were removed and the calls were disrupted with a Teflon homogenizer. The resultant was centrifuged (100,000 g, 1 hour), and the obtained supernatant was subjected to the Western blotting in the same manner as in (i). As a control, kidney cells from mice which were not treated with testosterone were subjected to the same operation. As a result, a band was detected for the cells obtained from mice treated with testosterone at the same position as the recombinant ODC, which band was thicker than the corresponding band detected in the control experiment (Fig. 5). An ODC band was also detected when the monoclonal antibody was used as the primary antibody.

11

(9) Immunostaining of Digestive Organ Tissues with Monoclonal Antibodies

Sections (paraffin‒embedded sections and frozen sections) were prepared from tissue specimens (formalin specimens and frozen specimens) of colon cancer and vicinities thereof obtained by surgery, and the sections were immunostained according to a conventional method (Extra Number of MEDICAL TECHNOLOGY, All about Staining Method, Ishiyaku Shuppan Co., Ltd., and Vector Stain ABC Elite Kit commercially available from Vector Co., Ltd.) using the purified monoclonal antibodies (3B‒10, 4B‒6) as the primary antibody. As a negative control, mouse IgG (commercially available from Cappel) having the same concentration as the primary antibody was used. The concentration of the primary antibody was 30 ‒ 40 ng/ml and the reaction was carried out at 30ºC for 30 minutes. As a result, although normal gland cells were not stained, the cancer gland cells were specifically stained. In the negative control experiment, neither the normal nor cancer cells were stained.

(10) Quantification of ODC Measured by Labelled Polyclonal Antibody

(i) Preparation of ODC Calibration Curve

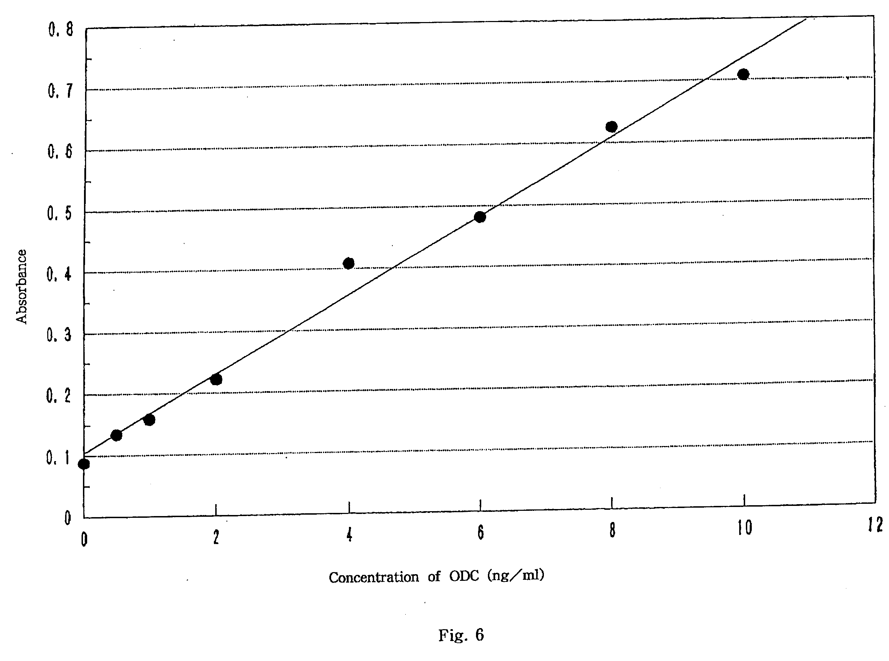

A calibration curve was prepared by sandwich ELISA using the ODC produced in (2), the polyclonal antibody and labelled polyclonal antibody produced in (7). That is, 50 $\mu$g/well of PBS containing 5 $\mu$g/ml of the polyclonal antibody was placed in wells of a 96‒well microtiter plate, and the antibody was adsorbed to the wells by incubation at 4ºC for 18 hours or at 30ºC for 2 hours. After washing the wells with PBS containing 0.5 ml/l of Tween 20 (PBS‒Tween), 320 $\mu$l/well of a blocking solution (Block Ace, commercially available from Dainippon Pharmaceutical, 4‒fold diluted) and the resultant was incubated at 4ºC for 18 hours or at 30ºC for 2 hours. Then 50 $\mu$l/well of ODC solution serially diluted with PBS‒Tween was added to the wells and the resultant was allowed to react at 30ºC for 2 hours. After the reaction, the wells were washed with PBS‒Tween, and 50 $\mu$l/well of PBS‒Tween containing 1 $\mu$g/ml of the biotin‒labelled polyclonal antibody was added. The resultant was allowed to react at 30ºC for 1 hour. After the reaction, the wells were washed with PBS‒Tween, and 100 $\mu$l/well of PBS‒Tween containing 5 $\mu$g/ml of avidin‒conjugated peroxidase (commercially available from Vector Co., Ltd) was added. The resultant was allowed to react at 30ºC for 30 minutes and the wells were washed with PBS‒Tween. Thereafter, 100 $\mu$l/well of 0.1 M citrate‒phosphate buffer (pH 5.0) containing 0.04% o‒phenylenediamine and 0.02% hydrogen peroxide was added and the resultant was allowed to react at 30ºC for 10 minutes. After stopping the reaction by adding 20 $\mu$l/well of 3.6 N sulfuric acid, the absorbances at 492 nm and at 630 nm were measured by a plate reader (MTP‒120, commercially available from Corona Co., Ltd). The results are shown in Table 2 and the obtained calibration curve is shown in Fig. 6.

Table 2

| ODC Concentration (ng/ml) | 0 | 0.5 | 1 | 2 | 4 | 6 | 8 | 10 |
|---|---|---|---|---|---|---|---|---|
| Absorbance | 0.088 | 0.134 | 0.158 | 0.221 | 0.408 | 0.481 | 0.625 | 0.707 |

(ii) Quantification of ODC in Cultured Human Cancer Cells and in Kidney of Mouse Induced by Testosterone

Using cultured human cancer cells (MKN − 28, COLO 205) and mouse kidney extract which mouse was treated with testosterone, ODC was quantified by the same method as in (i). The cultured human cancer cells were subjected to measurement after disrupting the cells by an ultrasonicator. The results are shown in Table 3 below.

Table 3

| | | ODC concentration (ng/mg Protein) |
|---|---|---|
| MKN − 28 | Medium Changed<br>Medium Not Changed | 5.5<br>4.8 |
| COLD 205 | Medium Changed<br>Medium Not Changed | 37<br>8.7 |
| Mouse Kidney Extract | Induced by Testosterone<br>Not Induced | 145<br>40 |

**Claims**

1.  A polyclonal antibody which specifically binds to human ornithine decarboxylase.

2.  A monoclonal antibody which specifically binds to human ornithine decarboxylase.

3.  The monoclonal antibody of claim 2, which is produced by hybridoma 3B − 10 (FERM BP − 4069).

4.  The monoclonal antibody of claim 2, which is produced by hybridoma 4B − 6 (FERM BP − 4070).

5.  A hybridoma which produces the monoclonal antibody of claim 2.

6.  The hybridoma of claim 8, which is hybridoma 3B − 10 (FERM BP − 4069).

7.  The hybridoma of claim 8, which is hybridoma 4B − 6 (FERM BP − 4070).

8.  A method for measuring human ornithine decarboxylase by an immunoassay utilizing the specific binding between the antibody of claim 1 and/or 2 and human ornithine decarboxylase.

9.  The method of claim 8, which comprises, in the order mentioned, the steps of:
    contacting a test sample possibly containing human ornithine decarboxylase with a first antibody immobilized in a carrier, which first antibody has a specific binding ability to human ornithine decarboxylase;
    washing off unbound components in said test sample;
    contacting a labelled second antibody having a specific binding ability to human ornithine decarboxylase with human ornithine decarboxylase in said test sample possibly bound to said carrier via said first antibody;
    washing off unbound second antibody; and
    measuring the label of said second antibody immobilized to said carrier.

10. A method for detecting cancer cells producing human ornithine decarboxylase, comprising im − munostaining the human ornithine decarboxylase with said antibody of claim 2.

factor X

Ile Glu Gly Arg ↓

ATC GAA GGT CGT   GGG ATC CCC GGG AAT TCA

BamHI Smal   EcoRI

protein A'   EcoRI   EcoRV

T   protein A'

λ P_R   pRIT2T   pBR322 ori

Amp^r

Scal   termination codon

GST   Tth111I

P tac   Amp^r

pGEX-3X

lacl^q   pBR322 ori

EcoRI

blunting

EcoRV

dephosphorization

preparation of Scal ~Tth111I fragment

blunting

ligation

factor X

Ile Glu Gly Arg ↓

ATC GAA GGT CGT   GGG ATC CCC GGG AAT TCA

BamHI Smal   EcoRI

termination codon

protein A'

T   protein A

λ P_R   pBR322 ori

pRIT2T-3X

Amp^r

FIG. 1

15

factor X

Ile Glu Gly Arg↓

ATC GAA GGT CGT  GGG ATC CCC GGG AAT TCA

BamHI SmaI  EcoRI

termination codon

protein A'

λ P_R

pRIT2T-3X

protein A

pBR322 ori

ODC (prepared by PCR)

BamHI
EcoRI

BamHI                    EcoRI

BamHI

EcoRI

ligation

protein A'        ODC

λ P_R

T

protein A

pBR322 ori

Amp^r

FIG.2

FIG. 3

Fig.4

Fig. 5

Fig. 6

EP 0 542 287 A2

```
GAATTCCTGG AGAGTTGCCT TTGTGAGAAG CTGGAAATAT TTCTTTCAAT TCCATCTCTT    60

AGTTTTCCAT AGGAACATCA AGAAATC ATG AAC AAC TTT GGT AAT GAA GAG TTT   114
                             Met Asn Asn Phe Gly Asn Glu Glu Phe
                                          90                  95

GAC TGC CAC TTC CTC GAT GAA GGT TTT ACT GCC AAG GAC ATT CTG GAC    162
Asp Cys His Phe Leu Asp Glu Gly Phe Thr Ala Lys Asp Ile Leu Asp
            100             105             110

CAG AAA ATT AAT GAA GTT TCT TCT TCT GAT GAT AAG GAT GCC TTC TAT    210
Gln Lys Ile Asn Glu Val Ser Ser Ser Asp Asp Lys Asp Ala Phe Tyr
        115             120             125

GTG GCA GAC CTG GGA GAC ATT CTA AAG AAA CAT CTG AGG TGG TTA AAA    258
Val Ala Asp Leu Gly Asp Ile Leu Lys Lys His Leu Arg Trp Leu Lys
    130             135             140

GCT CTC CCT CGT GTC ACC CCC TTT TAT GCA GTC AAA TGT AAT GAT AGC    306
Ala Leu Pro Arg Val Thr Pro Phe Tyr Ala Val Lys Cys Asn Asp Ser
145             150             155             160

AAA GCC ATC GTG AAG ACC CTT GCT GCT ACC GGG ACA GGA TTT GAC TGT    354
Lys Ala Ile Val Lys Thr Leu Ala Ala Thr Gly Thr Gly Phe Asp Cys
                165             170             175

GCT AGC AAG ACT GAA ATA CAG TTG GTG CAG AGT CTG GGG GTG CCT CCA    402
Ala Ser Lys Thr Glu Ile Gln Leu Val Gln Ser Leu Gly Val Pro Pro
            180             185             190

GAG AGG ATT ATC TAT GCA AAT CCT TGT AAA CAA GTA TCT CAA ATT AAG    450
Glu Arg Ile Ile Tyr Ala Asn Pro Cys Lys Gln Val Ser Gln Ile Lys
            195             200             205

TAT GCT GCT AAT AAT GGA GTC CAG ATG ATG ACT TTT GAT AGT GAA GTT    498
Tyr Ala Ala Asn Asn Gly Val Gln Met Met Thr Phe Asp Ser Glu Val
    210             215             220

GAG TTG ATG AAA GTT GCC AGA GCA CAT CCC AAA GCA AAG TTG GTT TTG    546
Glu Leu Met Lys Val Ala Arg Ala His Pro Lys Ala Lys Leu Val Leu
225             230             235             240

CGG ATT GCC ACT GAT GAT TCC AAA GCA GTC TGT CGT CTC AGT GTG AAA    594
Arg Ile Ala Thr Asp Asp Ser Lys Ala Val Cys Arg Leu Ser Val Lys
            245             250             255
```

F I G. 7

```
TTC GGT GCC ACG CTC AGA ACC AGC AGG CTC CTT TTG GAA CGG GCG AAA    642
Phe Gly Ala Thr Leu Arg Thr Ser Arg Leu Leu Leu Glu Arg Ala Lys
            260                 265                 270

GAG CTA AAT ATC GAT GTT GTT GGT GTC AGC TTC CAT GTA GGA AGC GGC    690
Glu Leu Asn Ile Asp Val Val Gly Val Ser Phe His Val Gly Ser Gly
            275                 280                 285

TGT ACC GAT CCT GAG ACC TTC GTG CAG GCA ATC TCT GAT GCC CGC TGT    738
Cys Thr Asp Pro Glu Thr Phe Val Gln Ala Ile Ser Asp Ala Arg Cys
            290                 295                 300

GTT TTT GAC ATG GGG GCT GAG GTT GGT TTC AGC ATG TAT CTG CTT GAT    786
Val Phe Asp Met Gly Ala Glu Val Gly Phe Ser Met Tyr Leu Leu Asp
305                 310                 315                 320

ATT GGC GGT GGC TTT CCT GGA TCT GAG GAT GTG AAA CTT AAA TTT GAA    834
Ile Gly Gly Gly Phe Pro Gly Ser Glu Asp Val Lys Leu Lys Phe Glu
            325                 330                 335

GAG ATC ACC GGC GTA ATC AAC CCA GCG TTG GAC AAA TAC TTT CCG TCA    882
Glu Ile Thr Gly Val Ile Asn Pro Ala Leu Asp Lys Tyr Phe Pro Ser
            340                 345                 350

GAC TCT GGA GTG AGA ATC ATA GCT GAG CCC GGC AGA TAC TAT GTT GCA    930
Asp Ser Gly Val Arg Ile Ile Ala Glu Pro Gly Arg Tyr Tyr Val Ala
            355                 360                 365

TCA GCT TTC ACG CTT GCA GTT AAT ATC ATT GCC AAG AAA ATT GTA TTA    978
Ser Ala Phe Thr Leu Ala Val Asn Ile Ile Ala Lys Lys Ile Val Leu
            370                 375                 380

AAG GAA CAG ACG GGC TCT GAT GAC GAA GAT GAG TCG AGT GAG CAG ACC   1026
Lys Glu Gln Thr Gly Ser Asp Asp Glu Asp Glu Ser Ser Glu Gln Thr
385                 390                 395                 400

TTT ATG TAT TAT GTG AAT GAT GGC GTC TAT GGA TCA TTT AAT TGC ATA   1074
Phe Met Tyr Tyr Val Asn Asp Gly Val Tyr Gly Ser Phe Asn Cys Ile
            405                 410                 415

CTC TAT GAC CAC GCA CAT GTA AAG CCC CTT CTG CAA AAG AGA CCT AAA   1122
Leu Tyr Asp His Ala His Val Lys Pro Leu Leu Gln Lys Arg Pro Lys
            420                 425                 430

CCA GAT GAG AAG TAT TAT TCA TCC AGC ATA TGG GGA CCA ACA TGT GAT   1170
Pro Asp Glu Lys Tyr Tyr Ser Ser Ser Ile Trp Gly Pro Thr Cys Asp
            435                 440                 445
```

F I G. 7 (continued)

```
GGC CTC GAT CGG ATT GTT GAG CGC TGT GAC CTG CCT GAA ATG CAT GTG  1218
Gly Leu Asp Arg Ile Val Glu Arg Cys Asp Leu Pro Glu Met His Val
    450             455             460

GGT GAT TGG ATG CTC TTT GAA AAC ATG GGC GCT TAC ACT GTT GCT GCT  1266
Gly Asp Trp Met Leu Phe Glu Asn Met Gly Ala Tyr Thr Val Ala Ala
465             470             475             480

GCC TCT ACG TTC AAT GGC TTC CAG AGG CCG ACG ATC TAC TAT GTG ATG  1314
Ala Ser Thr Phe Asn Gly Phe Gln Arg Pro Thr Ile Tyr Tyr Val Met
            485             490             495

TCA GGG CCT GCG TGG GAA CTC ATG CAG CAA TTC CAG AAC CCC GAC TTC  1362
Ser Gly Pro Ala Trp Glu Leu Met Gln Gln Phe Gln Asn Pro Asp Phe
        500             505             510

CCA CCC GAA GTA GAG GAA CAG GAT GCC AGC ACC CTG CCT GTG TCT TGT  1410
Pro Pro Glu Val Glu Glu Gln Asp Ala Ser Thr Leu Pro Val Ser Cys
        515             520             525

GCC TGG GAG AGT GGG ATG AAA CGC CAC AGA GCA GCC TGT GCT TCG GCT  1458
Ala Trp Glu Ser Gly Met Lys Arg His Arg Ala Ala Cys Ala Ser Ala
    530             535             540

AGT ATT AAT GTG TAG ATAGCAC TCTGGTAGCT GTTAACTGCA AGTTTAGCTT      1510
Ser Ile Asn Val Stop
545

GAATTAAGGG ATTTGGGGGG ACCATGTAAC TTAATTACTG CTAGTTTTGA AATGTCTTTG 1570

TAAGAGTAGG GTCGCCATGA TGCAGCCATA TGGAAGACTA GCATATGGGT CACACTTATC 1630

TGTGTTCCTA TGGAAACTAT TTGAATATTT GTTTTATATG GATTTTTATT CACTCTTCAG 1690

ACACGCTACT CAAGAGTGCC CCTCAGCTGC TGAACAAGCA TTTGTAGCTT GTACAATGGC 1750

AGAATGGGCC AAAAGCTTAG TGTTGTGACC TGTTTTTAAA ATAAAGTATC TTGAAATAAT 1810

TAGGC                                                            1815
```

F I G.  7 (continued)

23